# FASCICULE DE BREVET EUROPEEN

(11) **EP 1 023 893 B1**
(45) Date de publication et mention de la délivrance du brevet: **14.12.2005**
(21) Numéro de dépôt: 00400121.0
(22) Date de dépôt: 18.01.2000
(51) Int. Cl.: A61K 7/48, A61K 7/02, A61K 7/027, A61K 7/032, A61K 7/021

(54) **Composition de maquillage ou de soin contenant un organopolysiloxane hydrophile**
Schmink- oder körperpflegezusammensetzung mit einem hydrophilen organoppolysiloxan
Make up or personal care composition comprising an hydrophilic organopolysiloxane

(30) Priorité: 28.01.1999 FR 9900957
(43) Date de publication de la demande: 02.08.2000
(73) Titulaire: L'OREAL, 75008 Paris (FR)
(72) Inventeur: Bara, Isabelle, 75013 Paris (FR)
(74) Mandataire: Kromer, Christophe

(56) Documents cités:
- EP-A- 0 295 886
- EP-A- 0 381 166
- EP-A- 0 687 461
- EP-A- 0 790 055
- EP-A- 0 848 029
- EP-A- 0 850 643
- WO-A-98/00105
- US-A- 5 833 973
- PATENT ABSTRACTS OF JAPAN vol. 1998, no. 11, 30 septembre 1998 (1998-09-30) & JP 10 175816 A (TORAY DOW CORNING SILICONE CO LTD), 30 juin 1998 (1998-06-30)
- CHEMICAL ABSTRACTS, vol. 132, no. 7, 12 février 2000 (2000-02-12) Columbus, Ohio, US; abstract no. 83397, JINBO, KAZUKO ET AL: "Cosmetics controlling skin moisture-lipid balance" XP002136987 & JP 2000 007549 A (POLA CHEMICAL INDUSTRIES, INC., JAPAN) 11 janvier 2000 (2000-01-11)

## Description

La présente invention se rapporte à un procédé de maquillage matifiant de la peau et/ou des lèvres des êtres humains.

Les compositions de rouge à lèvres et de fond de teint connues comprennent généralement des corps gras tels que des huiles, des composés pâteux et des cires, ainsi qu'une phase particulaire généralement composée de charges et de pigments. Les charges servent généralement à modifier la texture de la composition ainsi qu'à matifier le film ou couche de composition déposé sur la peau et/ou les lèvres alors que les pigments servent généralement à apporter de la couleur à la composition.

L'effet de matité est particulièrement recherché pour les utilisateurs à peaux mixtes ou grasses, ainsi que sous les climats chauds et humides. Les charges matifiantes sont le plus souvent des charges absorbantes comme le talc, la silice, le kaolin ou des charges présentant des propriétés optiques de diffusion de la lumière, propriétés connues sous le nom d'effet "soft focus".

Plus récemment, on a utilisé des polymères matifiants (voir en particulier le document EP-A-790055) tels que des polymères siliconés réticulés connus sous les références commerciales KSG (KSG 6, 16, 17, 18) de la société Shin Etsu, Tréfils de la société Dow Corning ou Gransils pour la société Grand Industrie.

L'inconvénient de ces produits commerciaux est de contenir des huiles de silicones linéaires ou cycliques du type polydiméthylsiloxanes (PDMS) non réticulés et d'apporter un effet huileux, gras, sans effet frais, ce qui ne permet pas ou difficilement leur utilisation en milieu chaud et humide et/ou par les utilisateurs à peaux grasses. De plus ces produits commerciaux, même ceux exempts d'huile de silicone (Tréfils 505 C de Dow Corning par exemple), sont difficilement dispersibles dans un milieu aqueux. Ces produits sont présentés comme des polymères siliconés élastomèriques « insolubles dans l'eau » (voir notamment le document de Kao EP-A-0855178.

Ces polymères difficilement incorporables en phase aqueuse sont totalement hydrofuges. Du fait de leur forte incompatibilité avec l'eau et en particulier avec la sueur, cette dernière n'est pas absorbée par ces polymères et a même tendance à « perler » à la surface de la peau, lorsque celle-ci transpire. Le pouvoir matifiant de ces polymères a donc tendance à s'estomper au cours du temps.

Récemment, on a conçu des émulsions contenant ce type de polymère (cf. brevets US-A-5 421 004 de Kose et US-A-5 599 533 d'Estée Lauder) en vue d'améliorer leurs propriétés cosmétiques. Ces émulsions stables, bien qu'apportant moins de gras et plus de fraîcheur que les produits anhydres, perdent leur propriété matifiante initialement apportée par les polymères siliconés réticulés.

Bien qu'il existe des composés de types organosiloxanes réticulés se dispersant en milieu aqueux, comme par exemple, les composés de type KSG 20 ou KSG 21 vendus par la société Shin Etsu, et dont la structure chimique particulière est responsable de cette dispersion en milieu aqueux (présence de groupements polaires leur conférant des propriétés tensioactives), ces composés, contrairement à ceux de la composition de l'invention, n'apportent pas d'effet mat particulier.

Par ailleurs, les compositions de fond de teint et/ou de rouge à lèvres connues, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué sur la peau ou les lèvres nécessitant de renouveler régulièrement l'application de la composition de fond de teint ou de rouge à lèvres. Par ailleurs, l'apparition de ces traces inacceptables notamment sur les cols de chemisier peut écarter certaines femmes à utiliser ce type de maquillage.

Depuis plusieurs années, les cosméticiens se sont intéressés aux compositions de rouge à lèvres et de fond de teint « sans transfert ». Ainsi, la société Shiseido a envisagé dans sa demande de brevet JP-A-61-65809 des compositions de rouge à lèvres « sans transfert » contenant de 1 à 70% en poids d'une résine siloxysilicate (à réseau tridimensionnel) comportant des chaînes pendantes alkylées de 1 à 6 atomes de carbone ou phénylées, de 10 à 98% en poids d'une huile de silicone volatile à chaîne silicone cyclique et des charges pulvérulentes. De même la société Noevier à décrit dans le document JP-A-62-61911 des compositions de rouge à lèvres, d'eye liner, de fonds de teint « sans transfert » comportant une ou plusieurs silicones volatiles associées à une ou plusieurs cires hydrocarbonées.

Ces compositions, bien que tout à fait satisfaisantes pour la propriété de « sans transfert » présentent l'inconvénient de laisser sur les lèvres, après évaporation des huiles de silicone, un film qui devient inconfortable au cours du temps (sensation de dessèchement et de tiraillement), écartant un certain nombre de femmes de ce type de produit de maquillage. Pour améliorer le confort de ce type de composition, on pourrait ajouter des huiles non volatiles, mais dans ce cas on perdrait en efficacité « sans transfert ».

Plus récemment, la société Revlon a envisagé dans sa demande de brevet EP-A-602905 des rouges à lèvres « sans transfert » contenant une silicone volatile cyclique ou linéaire à chaînes méthyle pendantes et une résine de silicone comportant une chaîne estérifiée pendante ayant de 12 à 18 atomes de carbone. Le film de rouge à lèvres, restant sur les lèvres après évaporation de la silicone volatile, a encore l'inconvénient de manquer de confort à l'application et notamment d'être trop sec. Elle a, en outre, envisagé dans sa demande de brevet EP-A-709 083 des fonds de teint « sans transfert » contenant une silicone volatile et associée à une résine siloxysilicate. Ces fonds de teint présentent encore l'inconvénient d'être peu confortables et secs au cours du temps.

La présente invention a justement pour objet un procédé de maquillage matifiant selon la revendication 1.

Par « élastomérique» on entend un matériau souple, déformable ayant des propriétés viscoélastiques et notamment la consistance d'une éponge ou d'une sphère souple. Son modèle d'élasticité est tel que ce matériau résiste à la déformation et possède une capacité limitée à l'extension et à la contraction. Ce matériau est capable de retrouver sa forme originelle suite à un étirement. Cet élastomère est formé de chaînes polymériques de haut poids moléculaire dont la mobilité est limitée par un réseau uniforme de points de réticulation.

Les organopolysiloxanes élastomériques de la composition du procédé de l'invention présentent des propriétés de structurant de milieu aqueux et sont aptes à augmenter la viscosité de la phase aqueuse. Ils ne sont pas desséchants pour la peau et apportent de bonnes propriétés cosmétiques, notamment de douceur, de fraîcheur, de matité et de sans transfert. Ces nouveaux élastomères conduisent à des compositions confortables à l'application, de bon étalement, douces et non collantes au toucher. Ces propriétés cosmétiques sont dues, d'une part à la texture des organopolysiloxanes et, d'autre part à leurs propriétés comparables à celles de microéponges piégeant les milieux aqueux et en particulier ceux de la composition et ceux dus à la transpiration de la peau.

La composition du procédé de l'invention peut se présenter sous forme de pâte, de solide, de crème plus ou moins fluide. Elle peut être une émulsion huile-dans-eau ou eau-dans-huile plus ou moins fluide, un gel hydrophile, solide ou souple. Cette composition peut avoir l'aspect d'une lotion, d'un gel, d'une crème, d'un produit coulé et même se présenter sous forme d'aérosol.

Les organopolysiloxanes élastomériques utilisés dans l'invention sont des composés hydrophiles partiellement ou totalement réticulés et de structure tridimensionnelle. L'épaississement de la phase aqueuse par ces élastomères peut être totale ou partielle.

Les élastomères de l'invention se présentent sous forme de poudre ou de gel émulsionné contenant un organopolysiloxane élastomère de structure tridimensionnelle, dispersé dans de l'eau. La suspension des particules est homogène.

Les organopolysiloxanes élastomériques selon l'invention peuvent être choisis parmi les polymères réticulés décrits dans la demande JP-A-10/175816. Selon cette demande, ils sont obtenus par réaction d'addition et de réticulation, en présence d'un catalyseur en particulier du type platine, d'au moins :
- (a) un organopolysiloxane (i) ayant au moins deux groupes vinyliques en position α-ω de la chaîne siliconée par molécule ; et
- (b) un organosiloxane (ii) ayant au moins un atome d'hydrogène lié à un atome de silicium par molécule.

En particulier, l'organopolysiloxane (i) est choisi parmi les polydiméthylsiloxanes et est plus spécifiquement un α-ω-diméthylvinyl polydiméthylsiloxane

Les organopolysiloxanes élastomériques de la composition du procédé selon l'invention se présente sous forme de suspension aqueuse. Cette suspension peut être notamment obtenue comme suit :
- (a) mélange de l'organopolysiloxane (i) et de l'organosiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organosiloxane (ii) en émulsion en présence d'un catalyseur de platine.

L'eau est avantageusement ajoutée à une température de 40-60°C. Après l'étape (e), il est possible de sécher les particules obtenues, pour en évaporer toute ou partie de l'eau piégée.

Les organopolysiloxanes sont sous la forme de particules solides déformables hydrophiles ayant une certaine dureté, mesurable avec un duromètre Shore A (selon la norme ASTM D2240) à la température ambiante ou avec la méthode japonaise JIS-A. Cette dureté peut être mesurée sur un bloc d'élastomère préparé à cet effet comme suit : mélange de l'organopolysiloxane (i) et de l'organosiloxane (ii) ; élimination de l'air du mélange ; moulage et vulcanisation au four à 100°C pendant 30 minutes ; refroidissement à température ambiante puis mesure de la dureté. La densité est aussi déterminée sur ce bloc d'élastomère.

En particulier, la dureté Shore est inférieure ou égale à 80 et mieux inférieure à 65. Les organopolysiloxanes de la composition de l'invention sont par exemple ceux commercialisés sous les noms BY 29-122 et BY 29-119 par la société Dow-Corning Electric. On peut aussi utiliser un mélange de ces produits commerciaux. Un bloc d'élastomère selon le produit BY-29122 présente une dureté de 7 et selon le produit BY-29122 une dureté de 30. La densité est de 0,97 à 0,98.

De façon préférentielle, la poudre d'organopolysiloxane élastomérique est présente dans la composition à un taux de 1 à 99% et mieux de 5 à 70%, ce qui correspond à un taux de polymère en matière active de 0,5 à 65% en poids et mieux de 3 à 45%. Elle joue en fait le rôle d'une charge hydrodispersible.

En particulier, les particules d'organopolysiloxane élastomérique (en matière active) ont une taille allant de 0,1 à 500 µm et mieux de 3 à 200 µm. Ces particules peuvent être sphériques, plates ou amorphes avec, de préférence, une forme sphérique.

Ces particules d'organopolysiloxane, pour se disperser de façon stable dans l'eau, peuvent être associées à un ou plusieurs tensioactifs non ioniques, cationiques ou anioniques de HLB > 8. De préférence l'étape (c) est obtenue en présence d'un émulsifiant non ionique.

La proportion de tensioactifs est de préférence de 0,1 à 20 parties en poids pour 100 parties en poids de la composition d'organopolysiloxane élastomérique, et mieux, de 0,5 à 10 parties en poids (cf. description du document JP-A-10/175816).

A ces poudres d'organopolysiloxane élastomérique peuvent être associées des corps gras liquides à température ambiante, appelées huiles, tels que ceux décrits dans le document JP-A-10 /175816, des cires ou des gommes solides à température ambiante, des corps gras pâteux, d'origine animale, végétale, minérale ou synthétique, leurs mélanges ainsi que des poudres inorganiques telles que celles décrites dans ce document.

La phase grasse additionnelle peut être quelconque et contenir des produits fluides à température ambiante comme les huiles siliconées, fluorées, fluorosiliconées, hydrocarbonées éventuellement partiellement siliconées. Ces huiles peuvent être volatiles à température ambiante et pression atmosphérique. Par huile volatile, on entend en particulier une huile susceptible de s'évaporer, en moins d'une heure, au contact de la peau ou des lèvres. Ces huiles peuvent représenter de 0 à 80 % du poids total de la composition.

Comme huiles utilisables dans la composition du procédé de l'invention, on peut citer notamment :
- les huiles hydrocarbonées d'origine animale telles que le perhydrosqualène ;
- les huiles hydrocarbonées végétales telles que les triglycérides liquides d'acides gras, par exemple les huiles de tournesol, de maïs, de soja, de courge, de pépins de raisin, de sésame, de noisette, d'abricot, de macadamia, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel ;
- les huiles de formule R¹COOR² dans laquelle R¹ représente le reste d'un acide gras supérieur comportant de 7 à 19 atomes de carbone et R² représente une chaîne hydrocarbonée ramifiée contenant de 3 à 20 atomes de carbone comme par exemple l'huile de Purcellin ;
- les hydrocarbures linéaires ou ramifiés, d'origine minérale ou synthétique tels que les huiles de paraffine volatiles ou non et leurs dérivés, la vaseline, les polydécènes, le polyisobutène hydrogéné tel que le parléam ;
- les esters et les éthers de synthèse comme le myristate d'isopropyle, des octanoates, décanoates ou ricinoléates d'alcools ou de polyalcools ;
- des alcools gras comme l'actyl dodécanol ou l'alcool oléique ;
- les huiles fluorées partiellement hydrocarbonées et/ou siliconées comme celle décrites dans le document JP-A-2-295912 ;
- les huiles siliconées comme les polyméthylsiloxanes à chaîne siliconée linéaire ou cyclique, liquides ou pâteux à température ambiante, les phényl diméthicones, les phényl triméthicones et les polyméthylphénylsiloxanes ;
- leurs mélanges.

Avantageusement, la composition du procédé selon l'invention peut contenir des cires hydrocarbonées, fluorées ou siliconées ou leurs mélanges, qui peuvent être solides ou semi-solides (sous forme d'une pâte) à température ambiante. Ces cires peuvent être d'origine végétale, minérale, animale et/ou synthétique. En particulier, ces cires présentent une température de fusion supérieure à 25 °C et mieux supérieure à 45 °C.

Les cires siliconées peuvent être des cires comportant une structure siliconée et des motifs à une ou plusieurs chaînes alkyle ou alcoxy pendantes et/ou en bout de structure siliconée, ces chaînes étant linéaires ou ramifiées et comportant de 10 à 45 atomes de carbone. Ces cires sont appelées respectivement des alkyldiméthicones et des alcoxydiméthicones. Par ailleurs, ces chaînes alkyle peuvent comporter une ou plusieurs fonctions ester.

Comme autres cires utilisables dans le procédé de l'invention, on peut citer les cires d'origine animale comme la lanoline, la cire d'abeilles ; les cires d'origine végétale telles que la cire de Carnauba ou de Candellila ; les cires d'origine minérale, par exemple de paraffine, de lignite ou les cires microcristallines, la cérésine ou l'ozokérite ; les cires synthétiques comme les cires de polyéthylèn ; leurs mélanges.

Ces corps gras peuvent être choisis de manière variée par l'homme du métier afin de préparer une composition ayant les propriétés, par exemple de consistance ou de texture, souhaitées.

En particulier, la présence de cires permet d'assurer une bonne résistance mécanique, notamment lorsque la composition se présente sous la forme d'un stick.

D'une manière générale, la composition peut comprendre de 0 à 50% du poids total de la composition de cire et de préférence de 10 à 30%.

La composition du procédé de l'invention peut comprendre, en outre, au moins un ingrédient addititionnel usuellement utilisé dans le domaine concerné choisi parmi les antioxydants, les huiles essentielles, les conservateurs, les actifs cosmétiques ou dermatologiques comme les hydratants (glycérine), les vitamines, les acides gras essentiels et les filtres solaires lipophiles, les polymères liposolubles notamment hydrocarbonés tels que les polyalkylènes, les gélifiants de phase aqueuse, les gélifiants de phase grasse, les parfums, les tensioactifs et leurs mélanges.

Ces ingrédients additionnels peuvent être présents dans la composition selon les quantités habituellement utilisées et par exemple à raison de 0 à 20% du poids total de la composition et mieux de 0,1 à 10%.

Avantageusement, la composition du procédé de l'invention contient comme ingrédients additionnels un ou plusieurs gélifiants de phase aqueuse. Parmi les gélifiants de phase aqueuse utilisables selon l'invention, on peut citer : les gélifiants cellulosiques hydrosolubles tels que l'hydroxyéthylcellulose, la méthylcellulose, l'hydroxypropylcellulose et la carboxyméthylcellulose ; la gomme de guar ; la gomme de guar quaternisée ; les gommes de guar non-ioniques comprenant des groupements hydroxyalkyle en C₁-C₆; les gommes de xanthane, de caroube, de scléroglucane, de gellane, de rhamsan, de karoya ; les alginates, la maltodextrine, l'amidon et ses dérivés, l'acide hyaluronique et ses sels ; les argiles et notamment les montmorillonites, les hectorites ou bentones, les laponites ; les polymères à groupement carboxylique comme les acides polyacryliques réticulés au moins partiellement neutralisé tels que les «Carbopol» ou «Carbomer» de la Société Goodrich (Carbomer 980 par exemple neutralisé par de la triéthanolamine -TEA en abréviation-) ; les polymères poly(métha)crylates de glycéryle ; la polyvinylpyrrolidone ; l'alcool polyvinylique ; les polymères et les copolymères réticulés d'acrylamide ; les homopolymères réticulés de chlorure de méthacryloyloxyéthyltriméthylammonium ; les polyuréthanes associatifs et leurs mélanges.

Selon le procédé de l'invention, le gélifiant de phase aqueuse est de préférence choisi parmi la gomme de xanthane, les argiles (bentone ou laponite), les polyuréthanes associatifs, les épaississants cellulosiques, notamment l'hydroxyéthyl cellulose, et les acides polyacryliques réticulés au moins partiellement neutralisés.

Bien entendu l'homme du métier veillera à choisir les éventuels ingrédients additionnels complémentaires et/ou leur quantité de telle manière que les propriétés avantageuses de la composition du procédé selon l'invention ne soient pas, ou substantiellement pas, altérées par l'adjonction envisagée. En particulier, ces additifs ne devront pas nuire à l'homogénéité, la stabilité, le confort, la matité, la fraîcheur et au « non transfert » de la composition.

La composition du procédé selon l'invention se présente sous la forme d'un produit de maquillage de la peau, en particulier un fond de teint, un fard à joues ou à paupières, un eye-liner, un stick anti-cernes, ou de maquillage des lèvres comme un rouge à lèvres.

Bien entendu la composition du procédé de l'invention doit être cosmétiquement ou dermatologiquement acceptable, à savoir non toxique et susceptible d'être appliquée sur la peau (y compris l'intérieur des paupières) ou les lèvres d'être humains.

De façon préférentielle, la composition du procédé de l'invention comprendre une phase particulaire, généralement présente jusqu'à 60 % du poids total de la composition, de préférence de 5 à 35 %, et qui peut comprendre des pigments et/ou des nacres et/ou des charges habituellement utilisés dans les compositions cosmétiques. La composition du procédé selon l'invention peut comprendre une matière colorante, notamment des colorants solubles dans le milieu et notamment hydrosolubles ou liposolubles.

Par pigments, il faut comprendre des particules blanches ou colorées, minérales ou organiques, insolubles dans le milieu de la composition, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent à modifier la texture de la composition ainsi que l'effet de matité/brillance.

Les pigments peuvent être présents dans la composition à raison de 0 à 60 % du poids de la composition finale, et de préférence à raison de 4 à 25 %. Comme pigments minéraux utilisables dans l'invention, on peut citer les oxydes de titane, de zirconium ou de cérium ainsi que les oxydes de zinc, de fer ou de chrome et le bleu ferrique. Parmi les pigments organiques utilisables dans l'invention, on peut citer le noir de carbone, et les laques de baryum, strontium, calcium, aluminium et leurs mélanges.

Les nacres peuvent être présentes dans la composition à raison de 0 à 20 % du poids total de la composition, de préférence à un taux de l'ordre de 2 à 15 %. Parmi les nacres utilisables dans l'invention, on peut citer le mica recouvert d'oxyde de titane, d'oxyde de fer, de pigment naturel ou d'oxychlorure de bismuth tel que le mica titane coloré.

Les charges peuvent être présentes à raison de 0 à 35 % du poids total de la composition, de préférence 5 à 15 %. On peut notamment citer le talc, le mica, la silice, le kaolin, les poudres de Nylon ® (Orgasol ® notamment de chez Atochem) et de polyéthylène, le Téflon ®, l'amidon, le nitrure de bore, des microsphères de copolymères telles que l'Expancel ® (Nobel Industrie), le Polytrap ® (Dow Corning) et les microbilles de résine de silicone (Tospearl ® de Toshiba, par exemple) et leurs mélanges.

Les colorants hydrosolubles sont notamment le jus de betterave, le bleu de méthylène et peuvent représenter de 0 à 6 % du poids total de la composition.

La composition du procédé selon l'invention peut être fabriquée à froid ou par chauffage d'un ou plusieurs organopolysiloxanes élastomériques sous forme de poudre dispersée dans de l'eau, ajout d'un ou plusieurs pigments, d'une ou plusieurs charges et/ou d'un ou plusieurs autres additifs, ajout éventuel de la phase grasse à l'état liquide (notamment portée à la température de fusion des cires la plus élevée), puis émulsification si nécessaire.

Elle peut aussi être obtenue par extrusion comme décrit dans la demande EP-A-667 146. Ce procédé consiste à malaxer la pâte (cires + huiles + additifs + pigments) pendant le refroidissement pour créer dans la masse des zones d'écrasement de la pâte à l'aide d'un broyeur à cylindres ou d'un extrudeur-mélangeur à vis. Ce procédé permet l'obtention d'une composition sous forme de pâte molle.

L'invention a encore pour objet l'utilisation de particules d'organopolysiloxane solide élastomérique au moins partiellement réticulé en suspension dans une phase aqueuse dans une composition cosmétique de maquillage pour matifier la peau ou les lèvres.

L'invention est illustrée plus en détail dans les exemples suivants. Les pourcentages sont donnés en poids.

### Exemple 1 : ne faisant pas partie de l'invention telle que revendiquée.

### Réalisation d'un gel matifiant :

◆ Carbomer 980 0,3%
◆ TEA 0,3%
◆ Silicone BY 29-119 15%
◆ Conservateur qs
◆ Eau qsp 100%

### Résultat :

Obtention d'un gel très matifiant, d'une grande fraîcheur à l'application, de bonne tenue dans le temps et conservant un effet mat dans le temps, contrairement aux produits de l'art antérieur.

### Préparation :

On ajoute l'organopolysiloxane à l'eau à température ambiante ensuite on ajoute le gélifiant, le neutralisant puis les conservateurs et on mélange le tout sous agitation.

### Exemple 2 :

### Réalisation d'un fond de teint mat et frais :

◆ Silicone BY 29-122 70%
◆ Pigments (oxydes de fer) 7%
◆ Talc 10%
◆ Glycérine 5%
◆ Conservateur qs
◆ Eau qsp 100 %

### Résultat :

Obtention d'un fond de teint frais, très mat, de bonne tenue dans le temps.

### Préparation :

On prépare cette composition comme dans l'exemple 1.

### Exemple 3 :

Comparaison de l'effet matifiant de l'organopolysiloxane hydrophile (BY29-119) en dispersion aqueuse par rapport à un organopolysiloxane lipophile (KSG-16) en dispersion huileuse.

Ce test comparatif a été réalisé sur 28 femmes à peau grasse et brillante sur le front par demi-visage (une zone traitée du front contre une zone non traitée). La mesure de la brillance cutanée a été effectuée à l'aide d'un appareil du type Matidiag SEI-M-0029-MATI01 tel que décrit dans le document FR-A-2650890. Les mesures sont effectuées au temps To, T10 minutes, T1 heure et T3 heures.

| Traitements | T10 mintutes - T0 | T1 heure - T0 | T3 heures - T0 |
|---|---|---|---|
| BY-29-119 | - 39 % | - 36 % | -29 % |
| KSG 16 | - 19 % | - 17 % | - 14 % |

Il ressort clairement de ce tableau que l'organopolysiloxane hydrophile confère un aspect matifiant significativement supérieur à celui de l'organopolysiloxane hydrophile. Cette supériorité apparaît même de façon importante à l'oeil nu. En effet, plus la valeur mesurée est négative plus le produit est matifiant. En outre, cette matité tient bien dans le temps.

### Exemple 4 : Fond de teint

◆ Oxyde de titane (anatase non traité) 6,48%
◆ Amylopectine/amylose réticulé par épichlorhydrine 2,5 %
◆ Oxyde de fer jaune 1,07%
◆ Oxydes de fer brun, jaune 0,3 %
◆ Oxyde de fer noir 0,15%
◆ Eau déminéralisée stérilisée 20 %
◆ Talc 2,5 %
◆ Glycérine 4 %
◆ Nano-oxyde de titane traité dispersé dans l'eau à 40 % 14 %
◆ Propylène glycol 8 %
◆ Poly diméthylsiloxane oxyéthyléné à groupements phosphates (PM* : 4500) 1 %
◆ Poly diméthylsiloxane réticulé en dispersion dans eau/émulsifiant non ionique (BY-29-119) 40 %

* PM : poids moléculaire en poids.

### Contre-exemple : Fond de teint

◆ Oxyde de titane (anatase non traité) 6,48%
◆ Oxyde de fer jaune 1,07%
◆ Oxydes de fer brun, jaune 0,3%
◆ Oxyde de fer noir 0,15%
◆ Mélange de poly diméthylsiloxane réticulé et de cyclopenta diméthylsiloxane (6/94) (KSG15 de Shin Etsu) 92 %

La différence de quantité d'organopolysiloxane entre l'exemple 4 et le contre-exemple est liée à la faisabilité de la composition.

La composition de l'exemple 4 et de celle du contre-exemple on été appliquées sur des femmes par « demi-cou » (peau nue et peau traitée), ayant au préalable appliqué une crème hydratante (Hydrative de Lancôme). Après 10 minutes de séchage à l'air, une collerette en tissu a été appliquée durant 30 minutes. Le dépôt laissé sur la collerette a été ensuite observé à l'oeil nu. Des notes de 0 à 7 on été attibuées au dépôt. Plus la note est élevée plus la composition transfert.

La composition 4 a reçu la note moyenne de 3,8 et la composition du contre-exemple la note de 4,3. Cette différence est significative.

Par ailleurs, la prise à l'éponge de la composition de l'exemple 4 et son application sur la peau sont supérieures à celles de la composition du contre-exemple.

## Revendications

1. Procédé de maquillage matifiant de la peau ou des lèvres d'êtres humains comprenant l'application sur la peau ou les lèvres d'une composition, matifiante contenant comme épaississant des particules d'organopolysiloxane solide élastomérique au moins partiellement réticulé en suspension dans une phase aqueuse, et une phase particulaire choisie parmi les pigments, les nacres, les charges et leurs mélanges.

2. Procédé selon la revendication 1, **caractérisé en ce que** l'organopolysiloxane élastomérique est obtenu par réaction d'addition et de réticulation, en présence d'un catalyseur, d'au moins :
- un organopolysiloxane (i) ayant au moins deux groupes vinyliques en position α-ω de la chaîne siliconée par molécule ; et
- d'un organopolysiloxane (ii) ayant au moins un atome d'hydrogène lié à un atome de silicium par molécule.

3. Procédé selon la revendication 1 ou 2, **caractérisé en ce que** l'organopolysiloxane (i) est choisi parmi les polydiméthylsiloxanes.

4. Procédé selon l'une des revendications 1 et 2, **caractérisé en ce que** l'organopolysiloxane (i) est un α-ω-diméthylvinyl polydiméthylsiloxane.

5. Procédé selon l'une quelconque des revendications précédentes, **caractérisé en ce que** la suspension de particules d'organopolysiloxane est obtenue selon les étapes suivantes :
- (a) mélange de l'organopolysiloxane (i) et de l'organopolysiloxane (ii) ;
- (b) ajout de la phase aqueuse contenant un émulsifiant au mélange de l'étape (a) ;
- (c) émulsification de la phase aqueuse et dudit mélange ;
- (d) ajout d'eau chaude à l'émulsion de la phase (c) ; et
- (e) polymérisation de l'organopolysiloxane (i) et de l'organopolysiloxane (ii) en émulsion en présence d'un catalyseur de platine.

6. Procédé selon la revendication précédente, **caractérisé en ce que** l'étape (c) est obtenue en présence d'un émulsifiant non-ionique.

7. Procédé selon l'une quelconque des revendications précédentes, **caractérisé en ce que** les particules ont une taille allant de 0,1 à 500 µm et mieux de 3 à 200 µm.

8. Procédé selon l'une quelconque des revendications précédentes, **caractérisé en ce que** les particules d'organopolysiloxane présentent une dureté JIS-A inférieure ou égale à 80 et mieux inférieure à 65.

9. Procédé selon l'une quelconque des revendications précédentes, **caractérisé en ce que** la phase particulaire est présente à raison de 5 à 60 % du poids total de la composition, de préférence de 5 à 35 %

10. Procédé selon l'une quelconque des revendications précédentes, **caractérisé en ce que** la phase particulaire comprend des pigments.

11. Procédé selon l'une quelconque des revendications précédentes, **caractérisé en ce que** les pigments sont choisis parmi les oxydes de titane, les oxydes de zirconium, les oxydes de cérium, les oxydes de zinc, les oxydes de fer, les oxydes de chrome, le bleu ferrique, le noir de carbone, les laques de baryum, strontium, calcium, aluminium et leurs mélanges.

12. Procédé selon l'une quelconque des revendications précédentes, **caractérisé en ce que** les pigments sont présents dans la composition à raison de 4 à 60 % du poids de la composition finale, et de préférence à raison de 4 à 25 %.

13. Procédé selon l'une quelconque des revendications précédentes, **caractérisé en ce que** la composition comprend des nacres.

14. Procédé selon l'une quelconque des revendications précédentes, **caractérisé en ce que** les nacres sont présentes dans la composition à raison de 2 à 20 % du poids total de la composition, de préférence à raison de 2 à 15 %.

15. Procédé selon l'une quelconque des revendications précédentes, **caractérisé en ce que** la composition comprend des charges.

16. Procédé selon l'une quelconque des revendications précédentes, **caractérisé en ce que** les charges sont choisies parmi le talc, le mica, la silice, le kaolin, les poudres de Nylon ® , les poudres de polyéthylène, le Téflon ®, l'amidon, le nitrure de bore, les microbilles de résine de silicone, et leurs mélanges.

17. Procédé selon l'une quelconque des revendications précédentes, **caractérisé en ce que** les charges sont présentes à raison de 5 à 35 % du poids total de la composition, de préférence 5 à 15 %.

18. Procédé selon l'une quelconque des revendications précédentes, **caractérisé en ce que** la composition comprend des colorants hydrosolubles.

19. Procédé selon l'une quelconque des revendications précédentes, **caractérisé en ce que** la composition contient, en outre, une phase grasse contenant un corps gras choisi parmi les huiles, les cires, les gommes et les corps gras pâteux, d'origine animale, végétale, minérale ou synthétique et leurs mélanges.

20. Procédé selon l'une des revendications précédentes, **caractérisé en ce que** la composition comprend, en outre, un gélifiant de phase aqueuse.

21. Procédé selon la revendication précédente, **caractérisé en ce que** le gélifiant de phase aqueuse est choisi parmi la gomme de xanthane, les argiles, les polyuréthanes associatifs, les épaississants cellulosiques, et les acides polyacryliques réticulés au moins partiellement neutralisés et leurs mélanges.

22. Procédé selon l'une des revendications précédentes, **caractérisé en ce que** la composition contient une matière colorante.

23. Pocédé selon l'une des revendications précédentes, **caractérisé en ce que** la composition contient, en outre, au moins un ingrédient choisi parmi les conservateurs, les antioxydants, les parfums, les gélifiants de phase aqueuse, les tensioactifs, les actifs cosmétiques ou dermatologiques leurs mélanges.

24. Procédé selon l'une des revendications précédentes, **caractérisé en ce que** la composition se présente sous forme d'une composition de fond de teint, de fard à joues ou à paupières, d'un produit anti-cernes, d'un rouge à lèvres, d'un eye-liner.

25. Utilisation de particules d'organopolysiloxane solide élastomérique au moins partiellement réticulé en suspension dans une phase aqueuse dans une composition cosmétique de maquillage comprenant une phase particulaire choisie parmi les pigments, les nacres, les charges et leurs mélanges, pour matifier la peau ou les lèvres.

## Patentansprüche

1. Verfahren zum mattierenden Schminken der menschlichen Haut oder der menschlichen Lippen, das das Aufbringen einer mattierenden Zusammensetzung auf die Haut oder die Lippen umfasst, wobei die Zusammensetzung als Verdickungsmittel Partikel eines zumindest teilweise vernetzten, elastomeren, festen Organopolysiloxans in Suspension in einer wässrigen Phase und eine Partikelphase enthält, die unter den Pigmenten, Perlglanzpigmenten, Füllstoffen und deren Gemischen ausgewählt ist.

2. Verfahren nach Anspruch 1, **dadurch gekennzeichnet, dass** das elastomere Polysiloxan in Gegenwart eines Katalysators durch Addition und Vernetzung zumindest der folgenden Verbindungen hergestellt wird:
- eines Organopolysiloxans (i) mit mindestens zwei Vinylgruppen in α,ω-Stellung der Siliconkette pro Molekül; und
- eines Organopolysiloxans (ii) mit mindestens einem an ein Siliciumatom gebundenen Wasserstoffatom pro Molekül.

3. Verfahren nach Anspruch 1 oder 2, **dadurch gekennzeichnet, dass** das Organopolysiloxan (i) unter den Polydimethylsiloxanen ausgewählt ist.

4. Verfahren nach einem der Ansprüche 1 und 2, **dadurch gekennzeichnet, dass** das Organopolysiloxan (i) ein α,ω-Dimethylvinylpolydimethylsiloxan ist.

5. Verfahren nach einem der vorhergehenden Ansprüche, **dadurch gekennzeichnet, dass** die Suspension der Organopolysiloxanpartikel gemäß den folgenden Schritten hergestellt wird:
- (a) Mischen von Organopolysiloxan (i) und Organopolysiloxan (ii);
- (b) Zugabe der wässrigen Phase, die einen Emulgator enthält, zu dem Gemisch aus Schritt (a);
- (c) Emulgieren der wässrigen Phase und des Gemisches;
- (d) Zugabe von heißem Wasser zu der Emulsion der Phase (c); und
- (e) Polymerisation von Organopolysiloxan (i) und Organopolysiloxan (ii) in Emulsion in Gegenwart eines Platinkatalysators.

6. Verfahren nach dem vorhergehenden Anspruch, **dadurch gekennzeichnet, dass** Schritt (c) in Gegenwart eines nichtionischen Emulgators durchgeführt wird.

7. Verfahren nach einem der vorhergehenden Ansprüche, **dadurch gekennzeichnet, dass** die Partikel eine Größe von 0,1 bis 500 µm und besser 3 bis 200 µm aufweisen.

8. Verfahren nach einem der vorhergehenden Ansprüche, **dadurch gekennzeichnet, dass** die Organopolysiloxanpartikel eine JIS-A-Härte von 80 oder darunter und besser unter 65 aufweisen.

9. Verfahren nach einem der vorhergehenden Ansprüche, **dadurch gekennzeichnet, dass** die Partikelphase in einer Menge von 5 bis 60 % des Gesamtgewichts der Zusammensetzung und vorzugsweise 5 bis 35 % vorliegt.

10. Verfahren nach einem der vorhergehenden Ansprüche, **dadurch gekennzeichnet, dass** die Partikelphase Pigmente umfasst.

11. Verfahren nach einem der vorhergehenden Ansprüche, **dadurch gekennzeichnet, dass** die Pigmente unter den Oxiden von Titan, Zirconium, Cer, Zink, Eisen und Chrom, Eisenblau, Ruß und Lacken von Barium, Strontium, Calcium, Aluminium und deren Gemischen ausgewählt ist.

12. Verfahren nach einem der vorhergehenden Ansprüche, **dadurch gekennzeichnet, dass** die Pigmente in der Zusammensetzung in einer Menge von 4 bis 60 % des Gewichts der fertigen Zusammensetzung und vorzugsweise in einer Menge von 4 bis 25 % enthalten sind.

13. Verfahren nach einem der vorhergehenden Ansprüche, **dadurch gekennzeichnet, dass** die Zusammensetzung Perlglanzpigmente enthält.

14. Verfahren nach einem der vorhergehenden Ansprüche, **dadurch gekennzeichnet, dass** die Perlglanzpigmente in einer Menge von 2 bis 20 % des Gesamtgewichts der Zusammensetzung und vorzugsweise 2 bis 15 % in der Zusammensetzung enthalten sind.

15. Verfahren nach einem der vorhergehenden Ansprüche, **dadurch gekennzeichnet, dass** die Zusammensetzung Füllstoffe enthält.

16. Verfahren nach einem der vorhergehenden Ansprüche, **dadurch gekennzeichnet, dass** die Füllstoffe unter Talk, Glimmer, Kieselsäure, Kaolin, Nylon®-Pulvern, Polyethylenpulvern, Teflon®, Stärke, Bornitrid, Siliconharzmikrokugeln und deren Gemischen ausgewählt sind.

17. Verfahren nach einem der vorhergehenden Ansprüche, **dadurch gekennzeichnet, dass** die Füllstoffe in einer Menge von 5 bis 35 % des Gesamtgewichts der Zusammensetzung und vorzugsweise 5 bis 15 % vorliegen.

18. Verfahren nach einem der vorhergehenden Ansprüche, **dadurch gekennzeichnet, dass** die Zusammensetzung wasserlösliche Farbstoffe enthält.

19. Verfahren nach einem der vorhergehenden Ansprüche, **dadurch gekennzeichnet, dass** die Zusammensetzung ferner eine Fettphase enthält, die Fettsubstanzen aufweist, die unter den Wachsen, Gummis und pastösen Fettsubstanzen tierischer, pflanzlicher, mineralischer oder synthetischer Herkunft oder deren Gemischen ausgewählt ist.

20. Verfahren nach einem der vorhergehenden Ansprüche, **dadurch gekennzeichnet, dass** die Zusammensetzung außerdem einen Gelbildner für die wässrige Phase enthält.

21. Verfahren nach dem vorhergehenden Anspruch, **dadurch gekennzeichnet, dass** der Gelbildner für die wässrige Phase unter Xanthangummi, Tonen, assoziativen Polyurethanen, Verdickungsmitteln auf Cellulosebasis und zumindest teilweise neutralisierten, vernetzten Polyacrylsäuren und deren Gemischen ausgewählt ist.

22. Verfahren nach einem der vorhergehenden Ansprüche, **dadurch gekennzeichnet, dass** die Zusammensetzung ein Farbmittel enthält.

23. Verfahren nach einem der vorhergehenden Ansprüche, **dadurch gekennzeichnet, dass** die Zusammensetzung ferner mindestens einen Bestandteil enthält, der unter den Konservierungsmitteln, Antioxidantien, Parfums, Gelbildnern für die wässrige Phase, grenzflächenaktiven Stoffen, kosmetischen oder dermatologischen Wirkstoffen und deren Gemischen ausgewählt ist.

24. Verfahren nach einem der vorhergehenden Ansprüche, **dadurch gekennzeichnet, dass** die Zusammensetzung als Zusammensetzung für Make-up, Wangenrouge, Lidschatten, Produkte gegen Augenringe, Lippenstifte oder Eyeliner vorliegt.

25. Verwendung von Partikeln eines elastomeren, festen, zumindest teilweise vernetzten Organopolysiloxans in Suspension in einer wässrigen Phase in einer kosmetischen Zusammensetzung zum Schminken, die eine Partikelphase enthält, die unter den Pigmenten, Perlglanzpigmenten, Füllstoffen und deren Gemischen ausgewählt ist, um die Haut oder die Lippen zu mattieren.

## Claims

1. Method for making up the skin or lips of human beings in a mattifying fashion, comprising the application to the skin or lips of a mattifying composition comprising, as thickener, particles of at least partially crosslinked elastomeric solid organopolysiloxane in suspension in an aqueous phase and a particulate phase chosen from pigments, pearlescent agents, fillers and their mixtures.

2. Method according to Claim 1, **characterized in that** the elastomeric organopolysiloxane is obtained by an addition and crosslinking reaction, in the presence of a catalyst, of at least:
- one organopolysiloxane (i) having at least two vinyl groups in the α,ω-position of the silicone chain per molecule; and
- one organopolysiloxane (ii) having at least one hydrogen atom bonded to a silicon atom per molecule.

3. Method according to Claim 1 or 2, **characterized in that** the organopolysiloxane (i) is chosen from polydimethylsiloxanes.

4. Method according to either of Claims 1 and 2, **characterized in that** the organopolysiloxane (i) is an α,ω-dimethylvinylpolydimethylsiloxane.

5. Method according to any one of the preceding claims, **characterized in that** the suspension of organopolysiloxane particles is obtained according to the following stages:
- (a) mixing the organopolysiloxane (i) and the organopolysiloxane (ii);
- (b) adding the aqueous phase comprising an emulsifier to the mixture of stage (a);
- (c) emulsifying the aqueous phase and the said mixture;
- (d) adding hot water to the emulsion of stage (c); and
- (e) polymerizing the organopolysiloxane (i) and the organopolysiloxane (ii) as an emulsion in the presence of a platinum catalyst.

6. Method according to the preceding claim, **characterized in that** the stage (c) is obtained in the presence of a nonionic emulsifier.

7. Method according to any one of the preceding claims, **characterized in that** the particles have a size ranging from 0.1 to 500 µm and better still from 3 to 200 µm.

8. Method according to any one of the preceding claims, **characterized in that** the organopolysiloxane particles exhibit a JIS-A hardness of less than or equal to 80 and better still of less than 65.

9. Method according to any one of the preceding claims, **characterized in that** the particulate phase is present in a proportion of 5 to 60% of the total weight of the composition, preferably of 5 to 35%.

10. Method according to any one of the preceding claims, **characterized in that** particulate phase comprises pigments.

11. Method according to any one of the preceding claims, **characterized in that** the pigments are chosen from titanium oxides, zirconium oxides, cerium oxides, zinc oxides, iron oxides, chromium oxides, ferric blue, carbon black, barium, strontium, calcium and aluminium lakes, and their mixtures.

12. Method according to any one of the preceding claims, **characterized in that** the pigments are present in the composition in a proportion of 4 to 60% of the weight of the final composition and preferably in a proportion of 4 to 25%.

13. Method according to any one of the preceding claims, **characterized in that** the composition comprises pearlescent agents.

14. Method according to any one of the preceding claims, **characterized in that** the pearlescent agents are present in the composition in a proportion of 2 to 20% of the total weight of the composition, preferably in a proportion of 2 to 15%.

15. Method according to any one of the preceding claims, **characterized in that** the composition comprises fillers.

16. Method according to any one of the preceding claims, **characterized in that** the fillers are chosen from talc, mica, silica, kaolin, Nylon® powders, polyethylene powders, Teflon®, starch, boron nitride, silicone resin microbeads, and their mixtures.

17. Method according to any one of the preceding claims, **characterized in that** the fillers are present in a proportion of 5 to 35% of the total weight of the composition, preferably 5 to 15%.

18. Method according to any one of the preceding claims, **characterized in that** the composition comprises water-soluble dyes.

19. Method according to any one of the preceding claims, **characterized in that** the composition additionally comprises a fatty phase comprising a fatty substance chosen from oils, waxes, gums and pasty fatty substances of animal, vegetable, mineral or synthetic origin and their mixtures.

20. Method according to one of the preceding claims, **characterized in that** the composition additionally comprises a gelling agent for the aqueous phase.

21. Method according to the preceding claim, **characterized in that** the gelling agent for the aqueous phase is chosen from xanthan gum, clays, associative polyurethanes, cellulose thickeners and crosslinked poly(acrylic acid)s which are at least partially neutralized, and their mixtures.

22. Method according to one of the preceding claims, **characterized in that** the composition comprises a colouring material.

23. Method according to one of the preceding claims, **characterized in that** the composition additionally comprises at least one ingredient chosen from preservatives, antioxidants, fragrances, gelling agents for the aqueous phase, surfactants, cosmetic or dermatological active principles, and their mixtures.

24. Method according to one of the preceding claims, **characterized in that** the composition is provided in the form of a foundation, face powder or eyeshadow composition, of a concealer product, of a lipstick or of an eyeliner.

25. Use of particles of at least partially crosslinked elastomeric solid organopolysiloxane in suspension in an aqueous phase in a cosmetic make-up composition comprising a particulate phase chosen from pigments, pearlescent agents, fillers and their mixtures for mattifying the skin or lips.
